# FASCICULE DE BREVET EUROPEEN

(11) **EP 1 009 301 B1**
(45) Date de publication et mention de la délivrance du brevet: **16.04.2003**
(21) Numéro de dépôt: 98942771.1
(22) Date de dépôt: 24.08.1998
(51) Int. Cl.: A61B 17/32

(54) **DISPOSITIF DE PROTECTION POUR UN OUTIL COUPANT**
SCHUTZVORRICHTUNG FÜR SCHNEIDWERKZEUG
DEVICE FOR PROTECTING A CUTTING TOOL

(30) Priorité: 25.08.1997 FR 9710627
(43) Date de publication de la demande: 21.06.2000
(60) Demande divisionnaire: 02003942.6
(73) Titulaire: Mosseri, Raphael, 75008 Paris (FR)
(72) Inventeur: Mosseri, Raphael, 75008 Paris (FR)
(74) Mandataire: Abello, Michel
(86) Numéro de dépôt international: FR9801838
(87) Numéro de publication internationale: WO99009898

(56) Documents cités:
- EP-A- 0 583 992
- WO-A-90/11725
- WO-A-94/01152
- FR-A- 2 700 960
- US-A- 4 725 267
- US-A- 4 850 996
- US-A- 4 985 021
- US-A- 5 049 136
- US-A- 5 250 064
- US-A- 5 330 492
- US-A- 5 360 408
- US-A- 5 364 370
- US-A- 5 423 766

## Description

La présente invention concerne un dispositif de protection pour un outil coupant, notamment pour un instrument médical tel qu'un bistouri .

Dans le domaine médical, de nombreux instruments coupants sont utilisés par le personnel soignant, les infirmières, les médecins, les chirurgiens et toute personne environnante, qui peuvent se blesser au cours de leur manipulation.

On peut distinguer d'une part les risques et d'autre part les conséquences.

Les risques actuels encourus lors de l'utilisation d'un instrument coupant, tel qu'un bistouri (en dehors du geste opératoire), peuvent engendrer
- soit une plaie accidentelle de l'opérateur soit par lui-même soit par une tierce personne lors de la remise de l'instrument,
- soit une plaie de l'aide opératoire lors de la restitution de l'instrument,
- soit une déstérilisation du champ de table lorsque le bistouri est déposé sur la table d'instruments.

Les conséquences sont :
a) sur le plan général, pour le bistouri, un risque de transmission d'un agent infectieux, viral (virus Hépatite, virus HIV,...), bactérien ou parasitaire. Certaines atteintes peuvent être irréversibles ;
b) sur la plan anatomique et fonctionnel (plus particulièrement avec le bistouri), section des tendons, section d'un nerf ou d'un pédicule collatéral d'un doigt; le pronostic est parfois très réservé lors de l'association ou de l'étendue de ces lésions, engendrant des incapacités fonctionnelles qui peuvent être permanentes avec des conséquences sur le plan professionnel mais aussi sur le plan fonctionnel pour les gestes de la vie courante.

Tout accident entraîne sa déclaration avec la mise en place de tout un protocole qui peut s'étendre sur plusieurs années et l'indemnisation de cet accident peut grever de façon importante le budget de la Santé Publique.

Le problème lié à la protection des personnes utilisant des outils coupants est particulièrement sensible dans le monde médical car il peut avoir des conséquences dramatiques sur la santé humaine.

Toutefois, malgré la gravité de ce problème et l'existence de maladies très infectieuses depuis de nombreuses années, aucune solution fiable et efficace n'a encore été trouvée pour surmonter ces inconvénients.

On connaît par le brevet US-A-5 250 064 un dispositif conforme au préambule de la revendication 1.

La présente invention a donc pour but d'éliminer les inconvénients précités, de répondre aux exigences de la Santé Publique et de proposer un dispositif de protection générale pour un outil coupant, et plus particulièrement pour un instrument médical.

A cet effet, l'invention a pour objet un dispositif de protection d'un outil coupant selon la revendication 1.

L'invention comporte les caractéristiques additionnelles suivantes :
- chaque branche de la fourche s'étend dans un plan sensiblement parallèle au plan de la lame et présente un contour qui est au moins circonscrit au contour de la lame dans la position protégée ;
- le manche du bistouri et la fourche sont fonctionnellement reliés l'un à l'autre par une zone de matière présentant une élasticité propre à permettre un écartement/rapprochement relatif du manche et de la fourche par déformation élastique de cette zone, celle-ci servant également de moyen élastique de rappel ;
- le manche du bistouri et ladite fourche sont réalisés en une seule pièce et en une même matière ;
- le manche du bistouri et la fourche sont articulés entre-eux et le moyen élastique de rappel est constitué d'un ressort intercalé entre eux ;
- ladite fourche comporte des butées pour interdire le déplacement de la lame au-delà des positions protégées et de travail ;
- la fourche comporte un manche servant de butée de fin de course pour le manche du bistouri lorsque la lame est déplacée dans sa position de travail ;
- le manche de la fourche présente une section transversale en forme générale de U définissant un logement pour y loger le manche du bistouri lorsqu'il est amené dans la position de travail ;
- les branches de la fourche sont reliées par un pont de matière servant de butée de fin de course pour la manche du bistouri lorsque la lame est rappelée dans la position protégée ;
- la fourche présente sur ses deux faces latérales externes une forme concave pour servir de surface d'appui pour les doigts de l'utilisateur.

L'invention sera mieux comprise, et d'autres buts, détails, avantages et caractéristiques de celle-ci apparaîtront plus clairement au cours de la description explicative détaillée qui va suivre de plusieurs modes de réalisation particuliers actuellement préférés de l'invention, donnés uniquement à titre illustratif et non limitatif, en référence aux dessins schématiques annexés, dans lesquels :
- la figure 1 est une vue de dessus d'un dispositif de protection pour un bistouri, conformément à l'invention ;
- la figure 2 est une vue en coupe longitudinale suivant la ligne I-I de la figure 1, la lame du bistouri étant dans sa position protégée ;
- la figure 3 est une vue analogue à la figure 2, mais représentant la lame du bistouri dans sa position de travail ;
- les figures 4 et 5 sont des vues en coupe transversale respectivement suivant les lignes IV-IV de la figure 4 et V-V de la figure 3 ; et
- la figure 6 est une vue en perspective d'une variante de réalisation d'un dispositif de protection pour un bistouri.

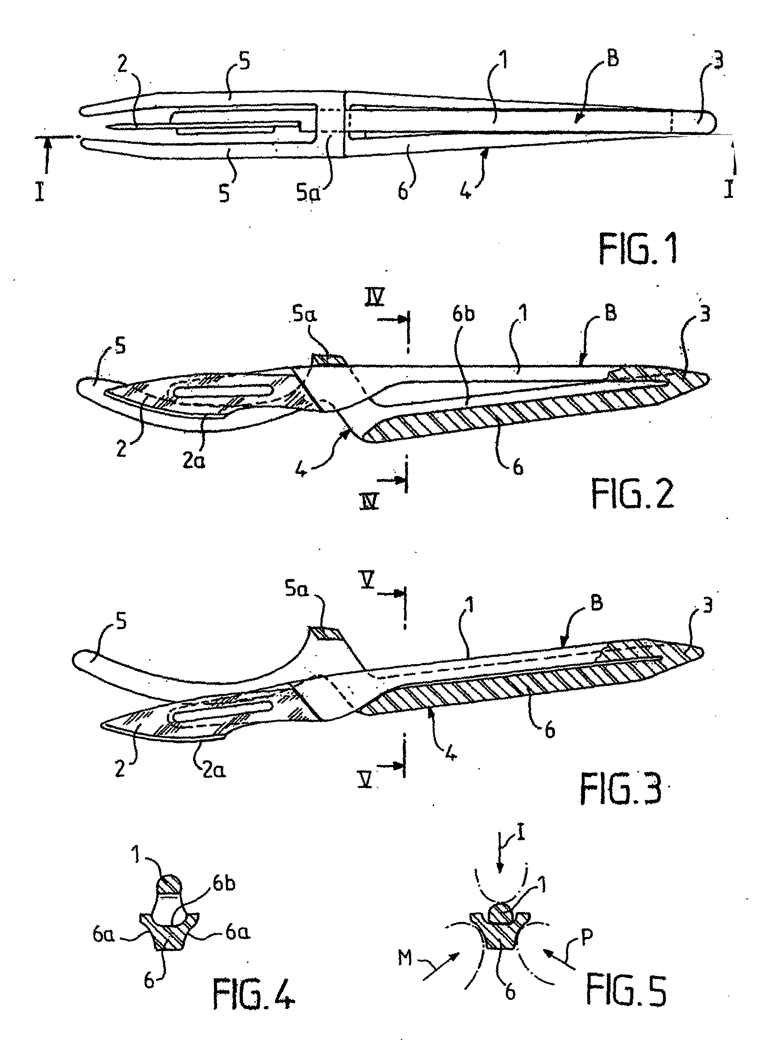

Suivant l'exemple particulier de réalisation représenté sur les figures 1 à 5, un premier mode de réalisation du dispositif de protection selon l'invention est adapté à un bistouri B. Le bistouri B, qui est connu en soi, comporte un manche 1 portant à sa portion distale une lame 2. Le manche 1 est relié à son extrémité proximale par une zone de liaison 3 à un élément en forme de fourche 4 servant à isoler la lame de l'extérieur. L'élément en forme de fourche 4 comporte deux branches sensiblement parallèles et espacées l'une de l'autre 5 qui sont reliées entre elles par un manche 6 formant la portion proximale de l'élément en forme de fourche 4. Le manche 1 du bistouri et l'élément en forme de fourche 4 peuvent être réalisés en une seule pièce et en une même matière, telle que le plastique. Ainsi, la zone de liaison 3 sert de "charnière" entre le manche 1 du bistouri et l'élément en forme de fourche 4 pour permettre un écartement/rapprochement relatif de ces derniers à la manière d'une pince à disséquer. L'élasticité propre de la matière au niveau de la zone de liaison 3 assurera automatiquement le rappel élastique de l'élément en forme de fourche 4 vers le manche 1 du bistouri B.

On pourrait également en variante, prévoir une articulation entre le manche 6 de la fourche 4 et le manche 1 du bistouri B, avec un ressort hélicoïdal de rappel monté sur l'axe de l'articulation ou un ressort à lame intercalé entre les deux manches. Dans ce cas, le manche 1 du bistouri B et l'élément en forme de fourche 4 pourraient être démontables et fabriqués séparément.

Comme il est visible sur les figures 4 et 5, le manche 6 de la fourche 4 présente une section transversale en forme générale de U pour y loger le manche 1 du bistouri B lorsque le dispositif est amené dans sa position de travail, comme illustré sur la figure 3. Le manche 6 de la fourche 4 présente sur ses deux faces latérales externes 6a une forme concave pour servir de surface d'appui pour le pouce P et le majeur M de la main d'un utilisateur, l'index I venant sur la partie supérieure du manche 1 du bistouri B, lorsque l'utilisateur saisit le dispositif à la manière d'un stylo. On notera que ce dispositif pourra aussi bien être utilisé par un droitier qu'un gaucher.

Comme mieux visible sur les figures 2 et 3, chaque branche 5 de la fourche s'étend dans un plan sensiblement parallèle au plan de la lame 2 du bistouri et de part et d'autre de celle-ci. Lorsque la lame 2 est dans sa position protégée représentée sur la figure 2, on voit que les branches 5 présentent, en projection perpendiculaire au plan de la lame, un contour externe qui enveloppe complètement le tranchant 2a de la lame. Les deux branches 5 de la fourche sont également reliées par un pont de matière Sa qui surmonte le manche 1 du bistouri B pour empêcher un écartement relatif supplémentaire des manches 1 et 6 à partir de la position protégée illustrée sur la figure 2. Autrement, il serait possible d'écarter vers le haut le manche 1 par rapport au manche 6 et d'introduire par exemple un doigt entre la lame 2 et les branches 5 de la fourche, lequel doigt pourrait être ainsi sectionné sous l'effet de la force de rappel élastique de la zone de liaison 3.

Le fonctionnement du dispositif illustré sur les figures 1 à 5 sera maintenant brièvement décrit.

Dans sa position de repos, le dispositif est tel que représenté sur la figure 2, c'est à dire avec la lame 2 du bistouri B qui est latéralement entourée par les branches 5 de la fourche, lesquelles branches 5 recouvrent aussi bien en longueur que en hauteur le tranchant 2a de la lame 2. Ainsi, la lame 2 est isolée de l'extérieur dans sa position protégée de repos. On pourrait prévoir également un moyen de verrouillage déverrouillable pour verrouiller le dispositif dans cette position.

Lorsque l'on veut dégager la lame 2 du dispositif, il suffit de placer ses doigts sur les manches 1 et 6 et de serrer les doigts, ce qui a pour effet de rapprocher les manches l'un contre l'autre jusqu'à ce que le manche 1 vienne en butée dans le logement 6b du manche 6. Dans la position illustrée sur la figure 3, les branches 5 de la fourche 4 se sont déplacées par rapport à la lame 2 découvrant ainsi la portion utile de la lame. Si le dispositif échappe par mégarde de la main de l'utilisateur, la lame sera automatiquement rappelée en position protégée sous l'effet de la force élastique de rappel de la zone de liaison 3.

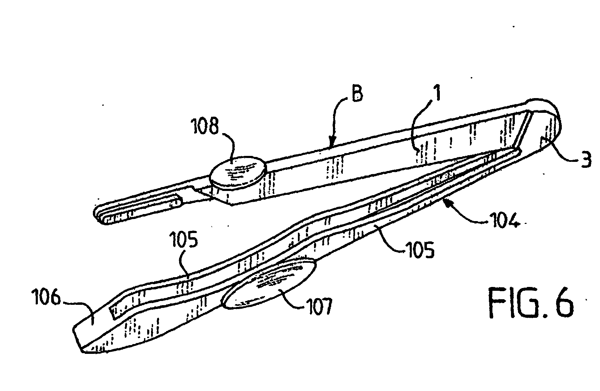

Sur la figure 6, on a représenté une variante de réalisation d'un dispositif de protection pour un bistouri B qui ne diffère du dispositif des figures 1 à 5 que par les caractéristiques suivantes :
- l'élément en forme de fourche 104 comporte deux branches sensiblement parallèles 105 espacées l'une de l'autre et reliées à une extrémité par la zone de liaison 3 et à leur extrémité libre opposée par un pont de matière 106 qui est destiné à servir de butée supérieure à la lame (non représentée sur la figure 6) du bistouri, pour empêcher un écartement relatif supplémentaire du manche 1 par rapport à l'élément en forme de fourche 104, les deux branches 105 étant également reliées sensiblement en leur milieu par une entretoise (non visible sur la figure 6), qui sert de butée inférieure pour le manche 1),
- une empreinte concave 107 est formée latéralement sur chaque branche 105 de l'élément en fourche 104 pour servir de surface d'appui pour les doigts, à la manière des faces 6a du mode de réalisation de la figure 4, et une empreinte concave 108 prévue sur le dessus du manche 1 pour servir de surface d'appui à l'index de la main de l'utilisateur.

Le fonctionnement de la variante illustrée sur la figure 6 est sensiblement analogue à celui du mode de réalisation représenté sur les figures 1 à 5.

Il est clair pour l'homme du métier que l'invention n'est limitée ni à la forme ni à la structure des dispositifs illustrés, toute autre forme ou structure permettant d'atteindre les mêmes fonctions faisant partie du cadre de la présente invention.

## Revendications

1. Dispositif de protection intégré pour un outil coupant (B), comportant
- un bistouri (B) constituant ledit outil coupant
- un moyen d'isolation (4, 104) pour isoler de l'extérieur une lame (2) formant la portion distale du bistouri, ce moyen étant relié à un manche (1) formant la portion proximale du bistouri avec une mobilité relative limitée et présentant une ouverture pour le passage de ladite lame entre une position protégée, dans laquelle ledit moyen entoure au moins partiellement ladite lame, et une position de travail, dans laquelle ladite lame est dégagée dudit moyen, et
- un moyen élastique (3) pour rappeler la lame dans la position protégée, ladite lame étant apte à se déplacer vers sa position de travail sous l'effet d'une pression externe (M, I, P) s'opposant à la force de rappel élastique, ledit moyen d'isolation ayant la forme générale d'une fourche (4, 104) dont les branches (5, 105) de la fourche définissent l'ouverture précitée pour le passage de la lame du bistouri, ledit moyen étant relié au manche (1) du bistouri de façon à permettre un déplacement de la fourche dans le plan de la lame, **caractérisé par le fait que** ladite fourche (4, 104) est reliée à l'extrémité proximale du manche (1) du bistouri par une zone de liaison (3) servant de charnière, pour permettre un rapprochement relatif du manche (1) et de la fourche (4, 104) lorsque l'utilisateur vient les serrer avec les doigts à la manière des branches d'une pince à disséquer, pour amener la lame dans sa position de travail.

2. Dispositif selon la revendication 1, **caractérisé en ce que** chaque branche (5, 105) de la fourche s'étend dans un plan sensiblement parallèle au plan de la lame et présente un contour qui est au moins circonscrit au contour de la lame dans la position protégée.

3. Dispositif selon la revendication 1 ou 2, **caractérisé en ce que** le manche (1) du bistouri et la fourche (4, 104) sont fonctionnellement reliés l'un à l'autre par une zone de matière (3) présentant une élasticité propre à permettre un écartement/rapprochement relatif du manche et de la fourche par déformation élastique de cette zone, celle-ci servant également de moyen élastique de rappel.

4. Dispositif selon la revendication 3, **caractérisé en ce que** le manche (1) du bistouri et ladite fourche (4, 104) sont réalisés en une seule pièce et en une même matière.

5. Dispositif selon la revendication 1 ou 2, **caractérisé en ce que** le manche (1) du bistouri et la fourche (4, 104) sont articulés entre-eux et le moyen élastique de rappel est constitué d'un ressort intercalé entre eux.

6. Dispositif selon l'une des revendications 1 à 5, **caractérisé en ce que** ladite fourche (4, 104) comporte des butées pour interdire le déplacement de la lame au-delà des positions protégées et de travail.

7. Dispositif selon la revendication 6, **caractérisé en ce que** la fourche (4) comporte un manche (6) servant de butée de fin de course pour le manche du bistouri lorsque la lame est déplacée dans sa position de travail.

8. Dispositif selon la revendication 7, **caractérisé en ce que** le manche (6) de la fourche (4) présente une section transversale en forme générale de U définissant un logement (6b) pour y loger le manche (1) du bistouri lorsqu'il est amené dans la position de travail.

9. Dispositif selon l'une des revendications 6 à 8, **caractérisé en ce que** les branches (5, 105) de la fourche sont reliées par un pont de matière (5a, 106) servant de butée de fin de course pour le manche du bistouri lorsque la lame est rappelée dans la position protégée.

10. Dispositif selon l'une des revendications précédentes, **caractérisé en ce que** la fourche (4, 104) présente sur ses deux faces latérales externes une forme concave (6a, 107) pour servir de surface d'appui pour les doigts (P, M) de l'utilisateur.

## Patentansprüche

1. Integrierte Schutzvorrichtung für ein Schneidewerkzeug (B), umfassend:
- ein Bistouri (B), das das Schneidewerkzeug bildet
- ein Isoliermittel (4, 104), um das Äußere der Klinge (2), die den distalen Teil des Bistouris bildet, zu isolieren, wobei dieses Mittel mit einem Griff (1), der den promixalen Teil des Bistouris bildet, mit einer eingeschränkten relativen Beweglichkeit verbunden ist und eine Öffnung für den Durchtritt der Klinge aus einer Schutzposition, in der das Mittel die Klinge zumindest teilweise umgibt, in eine Arbeitsposition, in der die Klinge von diesem Mittel freigegeben ist, aufweist, und
- ein Federmittel (3), um die Klinge in die Schutzposition zurückzubringen, wobei die Klinge so angepasst ist, dass sie unter der Einwirkung einer äußeren Kraft (M, 1, P), die der Federrückstellkraft entgegenwirkt, in ihre Arbeitsposition gebracht wird, wobei das Isoliermittel die allgemeine Form einer Gabel (4, 104) aufweist, deren Zinken (5, 105) die obengenannte Öffnung für den Übergang der Klinge des Bistouris definieren, und dieses Mittel mit dem Griff (1) des Bistouris derart verbunden ist, dass es eine Verschiebung der Gabel in der Ebene der Klinge gestattet, **dadurch gekennzeichnet, dass** die Gabel (4, 104) mit dem proximalen Ende des Griffs (1) des Bistouris über eine Verbindungszone (3) verbunden ist, die als Drehgelenk dient, um eine Annäherung zwischen dem Griff (1) und der Gabel (4, 104) zu ermöglichen, wenn der Benutzer diese mit den Fingern wie die Schenkel einer Sezierzange zusammendrückt, um die Klinge in ihre Arbeitsposition zu bringen.

2. Vorrichtung entsprechend Anspruch 1, **dadurch gekennzeichnet, dass** jede Zinke (5, 105) der Gabel sich in einer zur Ebene der Klinge im wesentlichen parallelen Ebene erstreckt und eine Kontur aufweist, die die Kontur der Klinge in der Schutzposition zumindest umschreibt.

3. Vorrichtung entsprechend Anspruch 1 oder 2, **dadurch gekennzeichnet, dass** der Griff (1) des Bistouris und die Gabel (4, 104) funktional über eine Materialzone (3) verbunden sind, die genau die Elastizität aufweist, um durch eine elastische Verformung dieser Zone eine Beabstandung/Annäherung zwischen Griff und Gabel zu ermöglichen, wobei diese Zone auch als Rückstellmittel dient.

4. Vorrichtung entsprechend Anspruch 3, **dadurch gekennzeichnet, dass** der Griff (1) des Bistouris und die Gabel (4, 104) einstückig und aus dem selben Material ausgeführt sind.

5. Vorrichtung entsprechend Anspruch 1 oder 2, **dadurch gekennzeichnet, dass** der Griff (1) des Bistouris und die Gabel (4, 104) zueinander beweglich sind und das Rückstellmittel von einer zwischen ihnen angeordneten Feder gebildet wird.

6. Vorrichtung entsprechend einem der Ansprüche 1 bis 5, **dadurch gekennzeichnet, dass** die Gabel (4, 104) Anschlagmittel aufweist, um eine Verschiebung der Klinge über die Schutz- und die Arbeitsposition hinaus zu verhindern.

7. Vorrichtung entsprechend Anspruch 6, **dadurch gekennzeichnet, dass** die Gabel (4) einen Griff (6) aufweist, der als Endanschlag für den Griff des Bistouris dient, wenn die Klinge in ihre Arbeitsposition gebracht wird.

8. Vorrichtung entsprechend Anspruch 7, **dadurch gekennzeichnet, dass** der Griff (6) der Gabel (4) einen allgemein U-förmigen Querschnitt aufweist, der einen Sitz (6b) für den Griff (1) des Bistouris ausbildet, wenn er in die Arbeitsposition gebracht wird.

9. Vorrichtung entsprechend einem der Ansprüche 6 bis 8, **dadurch gekennzeichnet, dass** die Zinken (5, 105) der Gabel über eine Materialbrücke (5, 106) miteinander verbunden sind, um als Endanschlag für den Griff des Bistouris zu dienen, wenn die Klinge in ihre Schutzposition zurückgebracht wird.

10. Vorrichtung entsprechend einem der vorangegangenen Ansprüche, **dadurch gekennzeichnet, dass** die Gabel (4, 104) an ihren äußeren Seitenflächen eine konkave Form (6a, 107) aufweist, um als Abstützfläche für die Finger (P, M) des Benutzers zu dienen.

## Claims

1. An integrated protective device for a cutting tool (B), comprising:
- a scalpel (B) constituting said cutting tool,
- an isolating means (4, 104) for isolating from the outside a blade (2) forming the distal portion of the scalpel, this means being connected to a handle (1) forming the proximal portion of the scalpel with alimited relative mobility and having an opening for said blade to pass between a protected position, in which said means surrounds said blade at least partially, and a working position, in which said blade is disengaged from said means, arid
- an elastic means (3) for returning the blade into the protected position, said blade being capable of moving towards its working position under the action of an external pressure (M, I, P) opposing the elastic return force, said isolation means being in the general form of a fork (4, 104) the branches (5, 105) of which define the aforementioned opening for the blade of the scalpel to pass through, said means being connected to the handle (1) of the scalpel so as to permit displacement of the fork in the plane of the blade, **characterised by** the fact that said fork (4, 104) is connected to the proximal end df the sleeve (1) of the scalpel by a connection zone (3) acting as a hinge, to permit a relative movement towards each other of the handle (1) and the fork (4, 104) when the user squeezes them with his fingers in the manner of the: arms of forceps, to bring the blade into its working position.

2. A device according to Claim 1, **characterised in that** each branch (5, 105) of the fork extends in a plane substantially parallel to the plane of the blade and has a contour which is at least circumscribed on the contour of the blade in the protected position.

3. A device according to Claim 1 or 2, **characterised in that** the handle (1) of the scalpel and the fork (4, 104) are functionally connected to each other by a zone of material (3) having an elasticity suitable to permit a movement away from and towards each other of the handle and the fork by elastic deformation of this zone, the latter also serving as an elastic return means.

4. A device according to Claim 3, **characterised in that** the handle (1) of the scalpel and said fork (4, 104) are made in a single piece and of one and the same material.

5. A device according to Claim 1 or 2, **characterised in that** the handle (1) of the scalpel and the fork (4, 104) are articulated together and the elastic return means is formed of a spring inserted between them.

6. A device according to one of Claims 1 to 5, **characterised in that** said fork (4, 104) comprises stops to prevent displacement of the blade beyond the protected and working positions.

7. A device according to Claim 6, **characterised in that** the fork (4) comprises a handle (6) acting as a stroke-end stop for the handle of the scalpel when the blade is displaced into its working position.

8. A device according to Claim 7, **characterised in that** the handle (6) of the fork (4) has a cross-section in a general U-shape defining a housing (6b) to accommodate the handle (1) of the scalpel when it is brought into the working position.

9. A device according to one of Claims 6 to 8, **characterised in that** the branches (5, 105) of the fork are connected by a bridge of material (5a, 106) acting as a stroke-end stop for the handle of the scalpel when the blade is returned into its protected position.

10. A device according to one of the preceding claims, **characterised in that** the fork (4, 104) has on its two outer lateral faces a concave shape (6a, 107) to act as a bearing surface for the fingers (P, M) of the user.
